# EUROPEAN PATENT SPECIFICATION

(11) **EP 4 613 222 B1**
(45) Date of publication and mention of the grant of the patent: **15.07.2026**
(21) Application number: 25161747.8
(22) Date of filing: 05.03.2025
(51) Int. Cl.: A61B 17/80

(54) **DUAL HEAD ELBOW PLATE**
ZWEIKOPF-ELLBOGENPLATTE
PLAQUE DE COUDE À DOUBLE TÊTE

(30) Priority: 08.03.2024 GR 20240100174; 01.05.2024 US 202418652290
(43) Date of publication of application: 10.09.2025
(73) Proprietor: Stryker European Operations Limited, Carrigtwohill, Co. Cork T45 HX08 (IE)
(72) Inventor: LOUVIS, Eleftherios, Carrigaline, P43FX98 (IE)
(74) Representative: Röthinger, Rainer

(56) References cited:
- WO-A1-2014/200233
- US-A1- 2007 233 114
- US-A1- 2010 069 966
- US-A1- 2014 031 877
- US-A1- 2019 321 193
- US-A1- 2023 044 693

## Description

### BACKGROUND OF THE INVENTION

Bone plates are commonly used for osteosynthesis applications, in particular for the treatment of bone fractures and bone stabilization. A typical fracture fixation procedure involves fixing a single bone plate to bone portions on either side of a fracture. A reduction of the fracture may also be performed, either utilizing secondary tools or through the use of compression holes in the bone plate. The latter may include the fixing of the bone plate on one side of the fracture and inserting a screw into a compression hole located on the opposite side. A typical compression hole is designed to cause a translation of the screw therein, which in turn translates the underlying bone portion to thereby reduce the fracture.

Certain fractures and/or anatomical features pose certain issues to this type of procedure. For instance, elbow fractures are often challenging for orthopedic surgeons to treat due to difficulty in achieving bone stabilization. A common surgical approach for bone plating to treat an elbow fracture includes a mid-line posterior incision that exposes the distal humerus medially, posteriorly, and laterally. Such an incision typically allows for the placement of two bone plates to stabilize the bone.

While using dual bone plates to treat elbow fractures is common, this dual plating still poses many challenges such as limited linear compression options, increased operation time, and excessive drilled holes and screws on the bone. Other drawbacks during operation include the crowding of many different plates in the surgical tray to ensure the plate required to fix the fracture is provided. Additionally, the overlap of independent plate shafts can limit some of the dual plating combinations e.g., a combination of a posterolateral plate and a posteromedial plate is not possible.

Unitary elbow plates used to treat elbow fractures are limited to specific configurations (*e.g.*, pure posterior shafts and articular extensions), and such plates lack the ability to allow multi-directional compressions along different axes. Many such plates also often lack the ability for variable angle screw placement. Consequently, these plates may not be an ideal option for bone stabilization.

WO 2014/200233 A1 discloses a calcaneus plate for treating the fracture of the calcaneus, and a calcaneus plate system. The disclosed calcaneus plate comprises first to fourth plate segments. The first plate segment is horizontally arranged. The second plate segment is upwardly extended in a vertical direction crossing a horizontal direction, from the back end of the first plate segment. The third plate segment is extended in a diagonal direction between the first plate segment and the second plate segment, from a point where the first plate segment and the second plate segment meet. The fourth plate segment is extended from the end of the third plate segment toward the front end of the first plate segment. The disclosed calcaneus plate system comprises: a calcaneus plate; and a locking screw to be fastened to the internal thread of the calcaneus plate. US 2010/0069966 A1 discloses a bone plate system for the internal fixation of metacarpal and phalanx fractures of the hand. The plates are structured to permit independent reconfiguration of holes of the plates relative to a longitudinal axis and are configured to orient fasteners to interdigitate with holes displaced along the longitudinal axis. The plates are very thin, and a locking screw with a low profile head design is provided for use therewith.

Thus, there is a need for a dual headed plate having advanced compression options with a faster and easier application than two independent plates that eliminates known drawbacks.

### BRIEF SUMMARY OF THE INVENTION

The present invention addresses the aforementioned drawbacks by providing a unitary dual headed plate with multi-directional compression along different axes. The unitary plate includes a Y-shaped plate with at least one compression hole on each of the three ends. These compression holes are configured to allow advanced compression options during reduction of the fracture, especially in the context of an elbow plate. The plates according to the present invention provide the surgeon with options heretofore not seen in elbow plates, including the ability to treat complex fractures.

The present invention is defined in claim 1 while preferred embodiments are set forth in the dependent claims. Associated surgical methods are also described herein to aid understanding the invention. These methods do not form part of the invention.

In accordance with an embodiment of the present disclosure, a bone plate for stabilizing a bone fracture includes: a unitary plate having a first portion, a second portion, and a third portion, each of the second and third portions being attached to the first portion and branching away from each other; at least one compression hole on each portion of the unitary plate; and compression holes that are configured to allow compression along three different directions, and the unitary plate is configured to allow multi-directional compression along the three different directions towards a single point.

In some embodiments, the first embodiment includes a Y-shaped unitary plate.

In a further embodiment, at least one compression hole is a locking compression hole.

In yet a further embodiment, the bone plate includes a non-locking hole.

In yet a further embodiment, the bone plate includes a locking hole.

In yet a further embodiment, the compression hole is configured to allow independent compression along the first portion, the second portion, and the third portion.

In yet a further embodiment, the locking compression hole is configured to accept the locking screw and the non-locking screw at varying angles with respect to a central axis of the hole.

In yet a further embodiment, the second portion and the third portion branch away from each other at an angle of 90 or 180 degrees.

In accordance with another embodiment of the present disclosure, a bone plating system for stabilizing a bone fracture includes: a single plate having a first portion, a second portion, and a third portion; at least one locking compression hole on each portion of the single plate; a non-locking bone screw capable of being received through one of the locking compression holes and into a bone; and a locking bone screw capable of being received through one of the locking compression holes and into the bone. The first portion splits into the second and third portions and the locking compression holes include longitudinal axes along a bone surface.

Embodiments of this second embodiment may include a single plate configured to allow multi-directional compression along the longitudinal axes on the bone surface.

In a further embodiment, the single plate is configured to allow multi-directional compression along the longitudinal axes on the bone surface at an angle of 50 to 160 degrees.

In yet a further embodiment, the single plate is configured to allow multi-directional compression along the longitudinal axes on the bone surface towards a single point on the bone.

In yet another embodiment, the single plate is configured to allow multi-directional selective compression and decompression along the longitudinal axes on the bone surface.

In yet another embodiment, the single plate is configured to allow varying compression length along the longitudinal axes on the bone surface.

In yet another embodiment, the single plate is configured to allow lagging of bone fragments in a crisscross function.

In accordance with an example useful for understanding the invention, a method of stabilizing a bone includes: placing a plate on a bone; inserting a first non-compression bone screw through a first hole in a second portion of the bone plate; inserting a second non-compression bone screw through a first hole in a third portion of the plate; inserting a first compression bone screw through a first hole in a first portion of the plate and compressing a first portion of the bone by threading the first compression bone screw into the bone; inserting a second compression bone screw through a second hole in the second portion of the plate, removing the first non-compression bone screw, and compressing the second portion of the bone by threading the second compression bone screw into the bone; and inserting a third compression bone screw through a second hole in the third portion of the plate, removing the second non-compression bone screw, and compressing the third portion of the bone by threading the third compression bone screw into the bone, wherein the non-compression bone screw includes a non-locking screw and a locking screw.

Examples of this third embodiment may include threading the compression bone screw into the bone at an angle through the locking compression hole.

In a further embodiment, the compression bone screw includes the non-locking bone screw.

In yet a further embodiment, the plate is manufactured via an additive manufacturing process.

In yet a further embodiment, the bone is a distal humerus.

### BRIEF DESCRIPTION OF THE DRAWINGS

A more complete appreciation of the subject matter of the present invention and of the various advantages thereof can be realized by reference to the following detailed description in which reference is made to the accompanying drawings in which:
FIG. 1A is a top view of a bone plate in accordance with one embodiment of the present invention.
FIG. 1B is a perspective view of the bone plate of FIG. 1A placed on a distal portion of a humerus bone.
FIG. 2 is a bottom view of the bone plate of FIG. 1A.
FIG. 3 is another perspective view of the bone plate of FIG. 1A.
FIG. 4A is a left side elevation view of the bone plate of FIG. 1A.
FIG. 4B is a right side elevation view of the bone plate of FIG. 1A.
FIG. 5A is a top view of the bone plate according to another embodiment of the present invention.
FIG. 5B is a perspective view of the bone plate of FIG. 5A placed on a distal portion of a humerus bone.
FIG. 6 is a top view of the bone plate according to another embodiment of the present invention showing multi-directional compression towards a single point.
FIG. 7 is a top view of the bone plate of FIG. 6 showing varying compression length.
FIG. 8A is a top view of the bone plate according to another embodiment of the present invention.
FIG. 8B is a perspective view of the bone plate of FIG. 8A placed on a distal portion of a humerus bone.
FIG. 9A is a top view of the bone plate according to another embodiment of the present invention.
FIG. 9B is a perspective view of the bone plate of FIG. 9A placed on a distal portion of a humerus bone.
FIG. 10A is a top view of the bone plate according to another embodiment of the present invention.
FIG. 10B is a perspective view of the bone plate of FIG. 10A placed on a distal portion of a humerus bone.
FIG. 11A is a top view of the bone plate according to another embodiment of the present invention.
FIG. 11B is a perspective view of the bone plate of FIG. 11A placed on a distal portion of a humerus bone.
FIG. 12A is a top view of the bone plate of FIG.11A showing the crisscross placement of screws to achieve a lagging function.
FIG. 12B is a perspective view of the bone plate of FIG. 12A placed on a distal portion of a humerus bone.

### DETAILED DESCRIPTION

In describing the preferred embodiments of the invention, specific terminology will be used for the sake of clarity. However, the invention is not intended to be limited to any specific terms used herein, and it is to be understood that each specific term includes all technical equivalents, which operate in a similar manner to accomplish a similar purpose. In the drawings and in the description which follows, the term "proximal" means closer to the heart and the term "distal" means more distant from the heart. The term "anterior" means towards the front part of the body or the face and the term "posterior" means towards the back of the body. The term "medial" means toward the midline of the body and the term "lateral" means away from the midline of the body. The term "posterolateral" means towards the back of the body and away from the midline of the body. The term "posteromedial" means towards the back of the body and towards the midline of the body.

Certain bone plates of different bone plating systems are disclosed herein. These plates are designed to be anatomically specific, thereby ensuring accurate placement on typical bones. Such plates have also been designed in various sizes to be useable in connection with as many differently sized patients as possible. The particular designs shown have been designed using Stryker Orthopedics Modeling and Analytics ("SOMA^{®}") software. This resulted in evidence-based screw hole placement designed to offer a wide range of trajectory options for particular anatomy, as well as anatomical fit, creating contoured plates with left and right specific options. It is contemplated to further refine bone plating system to include plates for a particular type of patient, for instance, male/female and ethnic-specific. It is also contemplated to provide patient-specific plates in accordance with the present invention.

While certain of the plates disclosed herein are shown in the accompanying drawings as being anatomically correct for placement on a specific bone (*e.g.* a left elbow), it is to be understood that the plates of the present invention could be designed for placement on a different bone (*e.g.,* a right elbow). For instance, the left and right versions of a plate according to the present disclosure may simply be mirror images of each other. Likewise, the plates disclosed in the present application can be tailored to fit on other bones entirely - *e.g.,* the radius, the ulna or another bone such as the tibia.

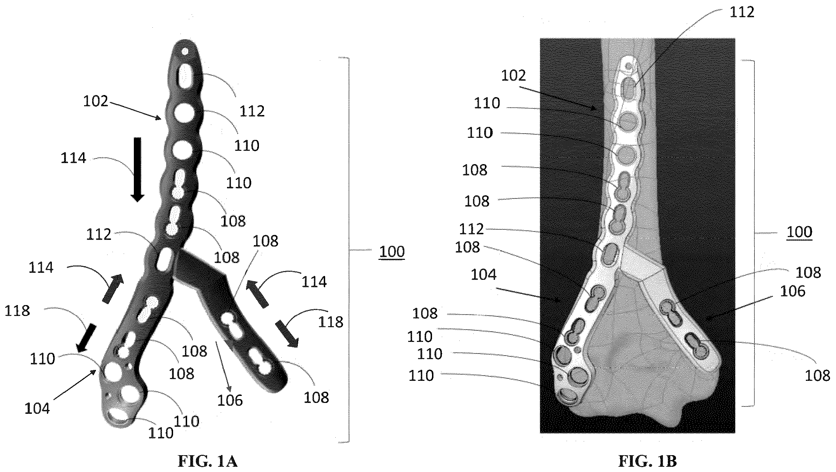

Referring to FIG. 1A, a bone plate according to an embodiment of the present disclosure includes a unitary plate 100 having a first portion 102, a second portion 104, and a third portion 106. First portion 102 is positioned over the posterior side of humerus as shown in FIG. 1B. Second portion 104 and third portion 106 are attached to first portion 102 and branch away from each other to define a Y-shaped plate 100. Each portion of plate 100 includes at least one compression hole. The inclusion of compression holes on each portion allows for compression along three different directions along a bone surface. This is particularly important for injuries like elbow fractures that include more than one fracture of the bone, which will be discussed in more detail below.

The plates of the present invention can include compression holes in any form as are known in the art. For instance, as shown in FIG. 1A, plate 100 may include compression holes in the form of a locking compression hole 108 or a non-locking compression hole 112. Both types of compression holes may be used to apply compression along bone surface to reduce a fracture, with locking compression holes 108 also providing a locking function if desired. In this way, these holes may accept both locking and non-locking screws. The inclusion of compression holes on each portion allows independent compression on bone surface along first portion 102, second portion 104, and third portion 106 of unitary plate 100. Indeed, plate 100 includes multiple compression holes on each portion that are oriented opposite to each other. Depending upon what type of injury is being treated, a surgeon can select one or more compression holes to properly reduce fracture(s) of the bone.

Bone plate 100 may also include one or more fixation holes, such as non-locking holes 110. In other embodiments, these fixation holes could be locking holes or any other type of hole. Moreover, plates in accordance with the present invention may include any combination of the compression and fixation holes. In one embodiment, non-locking screws can be angled (*e.g.,* up to 15 degrees in each direction for a total range of 30 degrees) to provide the flexibility to position screws based on patient needs. Locking holes may receive bone screws with threaded heads that engage threads in locking hole as to lock screw into plate. These fixation holes could also be designed such that both locking and non-locking screws can be received in a single hole. For instance, fixation holes in accordance with the present invention may be like those disclosed in U.S. Patent Nos. 6,322,562 and 11,039,865. Of course, other designs are also contemplated outside of just those patents. Locking compression holes 108 may include a similar fixation hole portion as do non-locking holes 110.

The dual headed plate of the present disclosure may be applied as treatment of distal humeral fractures as well as other joints. For instance, it is also contemplated to utilize similar plate designs in connection with distal femur or proximal tibia fractures. Plates in accordance with the present invention may be manufactured via an additive manufacturing process and specific designs can be based upon a specific patient. For instance, preoperative scans of a bone to be treated with a plate of the present invention can be utilized to generate patient-specific designs directed to the specific procedure to be performed. This can include not only plate shape, but also screw hole placement for optimal fracture fixation. Moreover, each portion of plates in accordance with the present invention can be designed to sit on different portions of the bone to be repaired. For instance, FIGS. 1B, 5B, 8B, 9B, 10B, and 11B show unitary plates placed on a distal portion of a humerus bone. In this regard, it is contemplated for plates in accordance with the present invention to be designed for placement in specific orientations (*e.g.*, anterior, medial or lateral). For instance, FIGS. 1A, 5A, and 6 show unitary plates intended for placement in posterior, posterolateral, and posteromedial positions.

Reference will now be made to various embodiments of bone plates of the present disclosure. Wherever possible, the same or like reference numbers will be used throughout the drawings to refer to the same or like features within a different 100-series of numbers. The differences between different embodiments will be focused on below, with the understanding that other portions of the plate may be similar or even identical.

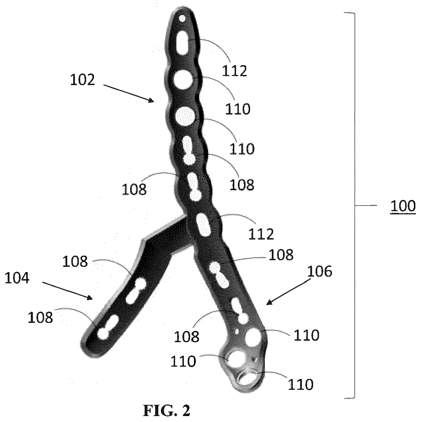

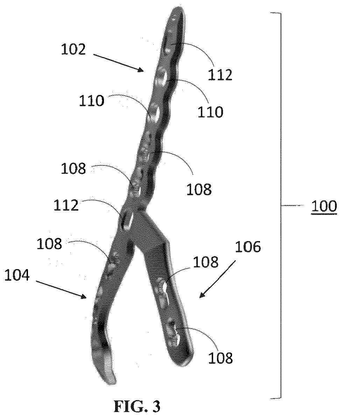

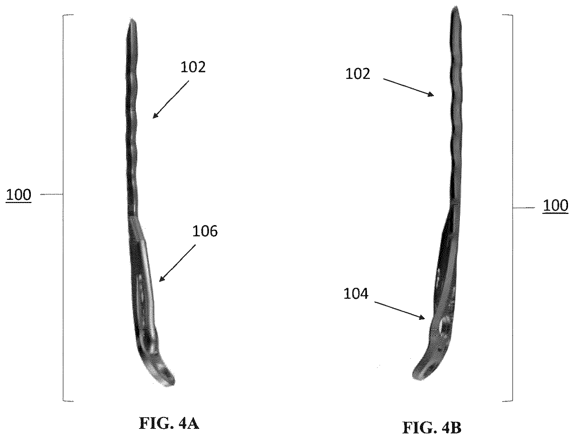

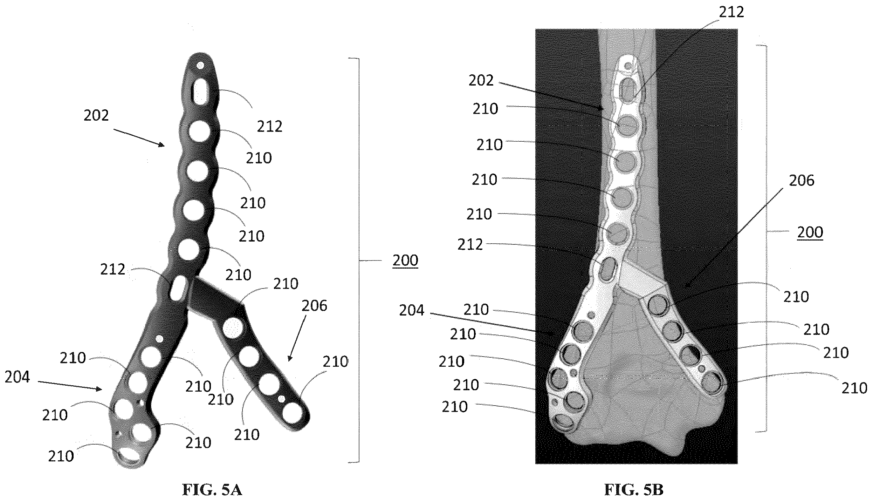

Beginning with FIG. 1A, plate 100 includes a combination of posterior 102, posterolateral 104, and posteromedial 106 portions. Whereas, independent posterolateral and posteromedial plates are unable to be combined due to shaft overlap, plate 100 makes this combination is possible. As shown in FIG. 1A, plate 100 includes at least two locking compression holes 108 on each portion of plate. Plate 100 also includes at least one of or a combination of non-locking holes 110, locking holes, or elongate 112 holes. Plate 100 allows selective multi-directional compression along longitudinal axes 114 and/or decompression along longitudinal axes 118 on bone surface as needed to reduce fractures and restore normal patient anatomy. Longitudinal axes 114 show different directions along each portion of plate 100 which allow for reduction of multiple bone fragments. This is due to the inclusion of differently oriented compression holes 108. Thus, when plate 100 is initially held into place via initial non-locking or locking screws, multi-directional compression and/or decompression can be applied via screws inserted in different locking compression holes 108. Further locking and non-locking screws can then be inserted into plate 100 to hold bone fragments in place after reduction is complete. FIG. 1B illustrates plate 100 in place on a distal portion of the humerus, while FIGS. 2-4B show different views of the plate. FIG. 5A shows plate 200, which is similar to that of FIG. 1A. In particular, plate 200 includes both non-locking holes 210 and elongate holes 212. Elongate holes 212 may also be used to apply compression along bone surface.

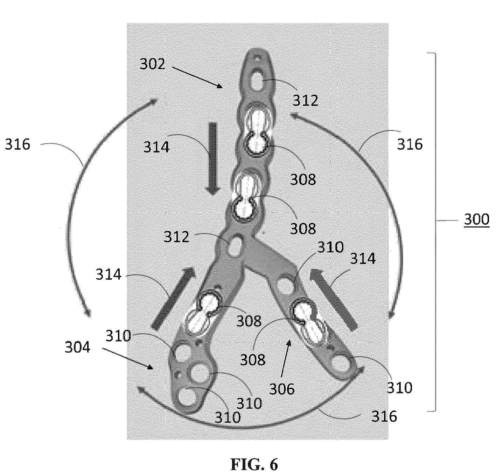

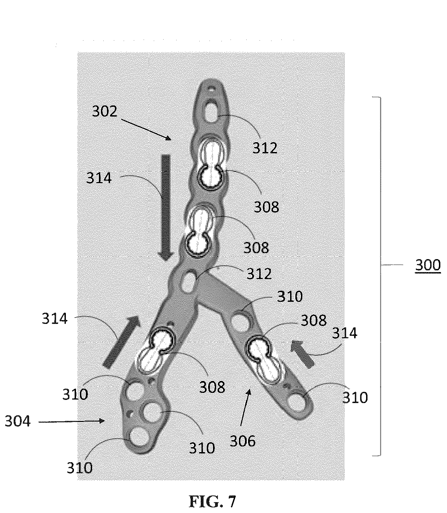

A further plate 300 is shown in FIG. 6, the design of which is also similar to that of FIG. 1A. However, plate 300 includes a single locking compression hole 308 on each of portions 304, 306. First portion 302, on the other hand, includes two locking compression holes 308. As each locking compression hole 308 faces in the same direction, they allow for multi-directional compression along longitudinal axes 314 on bone surface when screws are inserted through holes and into bone to reduce bone fragments. Plate 300 also allows multi-directional compression along longitudinal axes 314 on bone surface at projected angles 316 ranging from 50 to 160 degrees on a projection plane along posterior view of bone. As shown in FIG. 6, plate 300 may also allow multi-directional compression along longitudinal axes 314 on bone surface toward a single point, region of clinical importance, or compression towards a metaphyseal area of bone to reduce bone fragments. Multi-directional compression applied on bone surface may be equal along each longitudinal axis 314. As shown in FIG. 7, plate 300 also may allow varying multi-directional compression along longitudinal axes 314 on bone surface as appropriate for proper reduction of bone fragments. Multi-directional compression applied on bone surface may be different along each longitudinal axis 314.

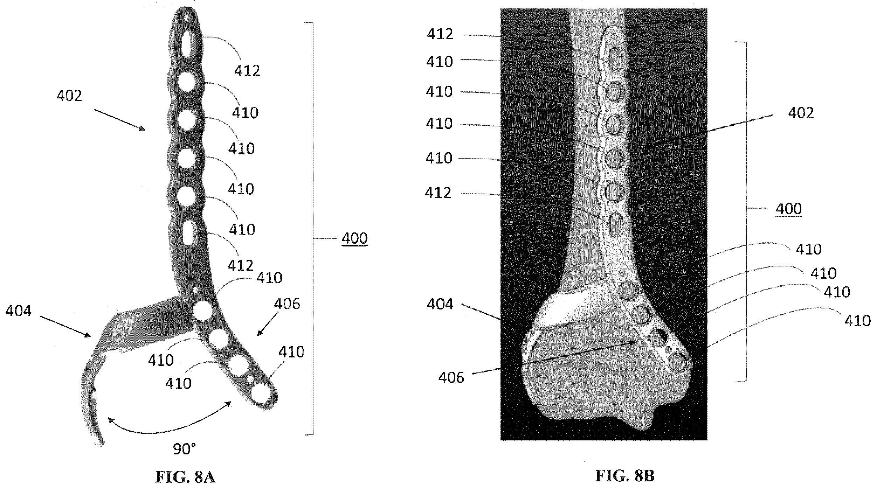

It is possible to vary the design of first, second and third portions of plates in accordance with the present disclosure to allow for reduction of complex bone fractures that previously could have only been accomplished using dual plating systems. As shown in FIG. 8A, plate 400 includes first portion 402, second portion 404, and third portion 406. Second portion 404 and third portion 406 are attached to first portion 402 and branch away from each other in a Y-shape in a similar fashion as in the above-discussed embodiments. First portion 402 is positioned over the posterior side of humerus. Second portion 404 and third portion 406 of plate 400 branch away from each other at a 90 degree angle. Second portion 404 and third portion 406 are positioned in a perpendicular configuration on bone. As such, plate 400 includes coverage of posteromedial and lateral portions of bone when reducing bone fragments. FIG. 8B illustrates plate 400 on a distal portion of a humerus bone.

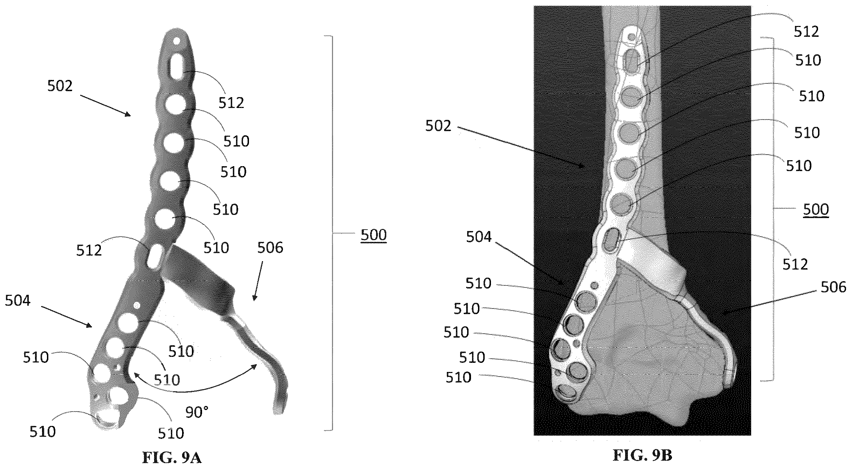

As shown in FIG. 9A, the design of plate 500 is similar to that of FIG. 8A. First portion 502 is positioned over posterior side of humerus. Second portion 504 and third portion 506 of plate 500 branch away from each other at a 90 degree angle. Second portion 504 and third portion 506 are positioned in a perpendicular configuration on bone. As such, plate 500 includes coverage of posterolateral and medial portions of bone when reducing bone fragments. FIG. 9B illustrates plate 500 on a distal portion of a humerus bone.

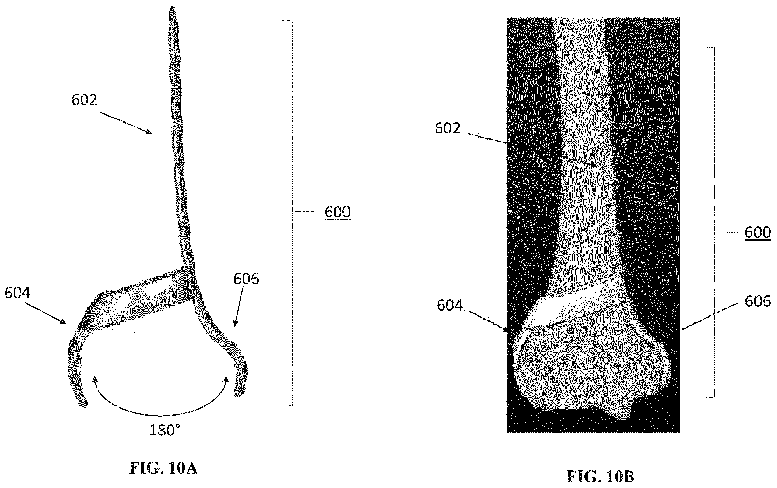

As shown in FIG. 10A, the design of plate 600 is also similar to that of FIG. 8A. First portion 602 is positioned over medial side of humerus. Second portion 604 and third portion 606 of plate 600 branch away from each other at a 180 degree angle. Second portion 604 and third portion 606 are positioned in a parallel configuration on bone. As such, plate 600 includes coverage of medial and lateral portions of bone when reducing bone fragments. FIG. 10B illustrates plate 600 on a distal portion of a humerus bone.

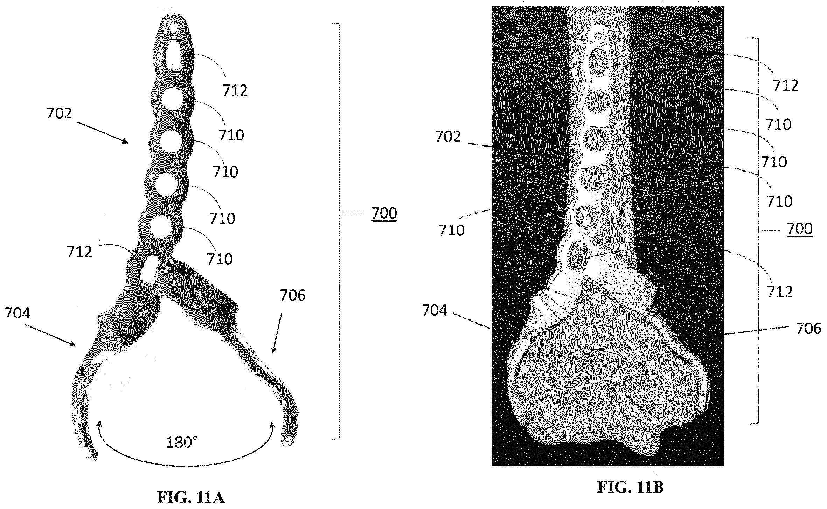

As shown in FIG. 11A, the design of plate 700 is also similar to that of FIG. 8A. First portion 702 is positioned over the posterior side of humerus. Second portion 704 and third portion 706 of plate 700 branch away from each other at a 180 degree angle. Second portion 704 and third portion 706 are positioned in a parallel configuration on bone. As such, plate 700 includes coverage of posterior, lateral, and medial portions of the bone when reducing bone fragments. FIG. 11B illustrates plate 700 on a distal portion of a humerus bone.

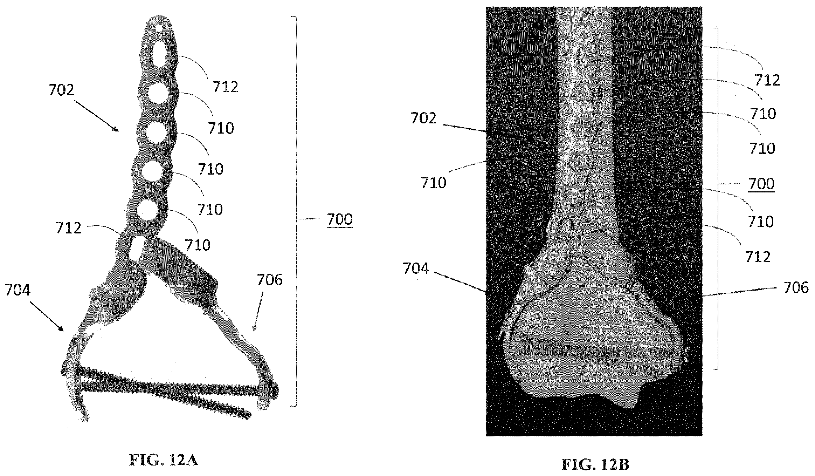

As shown in FIG. 12A, plate 700 includes holes that allow for lagging of bone fragments in a crisscross function rather than a traditional single lagging originating from a linear plate. Crisscross lagging of bone fragments allows multiple bone fragments to be compressed together by inserting screws through each fragment at an angle. For instance, medial and lateral fragments are compressed towards lateral and medial distal portions of the plate respectively. In one embodiment, non-locking screws may be inserted into non-locking holes 710 at an angle in a range of 30 degrees on second portion 704 and third portion 706 of plate 704. FIG. 12B illustrates plate 700 on distal portion of humerus bone showing crisscross lagging of bone fragments. Crisscross lagging of bone fragments requires direct lateral and direct medial distal plate portions as shown in bone plate 700 and bone plate 600. Bone plate 600 of FIG. 10A also includes non-locking holes 610 on second portion 604 and third portion 606 that allow for lagging of bone fragments in a crisscross function.

In accordance with another embodiment, of the present disclosure, a bone plating system includes a single plate including a first portion, a second portion, and a third portion. First portion splits into second and third portion forming Y-shaped single plate. Each portion of single plate includes at least one locking compression hole. Bone plating system also includes a non-locking bone screw and a locking bone screw capable of being received through locking compression holes and threaded into bone.

In accordance with an example useful for understanding the invention, a method of stabilizing a bone includes placing a bone plate on bone, inserting locking or non-locking screws into bone plate, inserting compression screws into bone plate, and threading compression screws into bone. Bone compression occurs by tightening compression screws in first, second, and third portion of plate. During compression of each plate portion the other two portions are firmly mounted to bone by at least one locking or non-locking screw. Any locking or non-locking screws that were previously added in portion of plate are removed before tightening compression screw in same portion to allow bone compression. The sequence of inserting and threading compression screws depends on which portion of bone needs to be compressed first to address bone fracture. Bone screws may be threaded into bone at an angle through locking compression holes, locking holes, non-locking holes, and elongate holes.

In a specific example useful for understanding the invention, the method may include inserting locking or non-locking screws into second and third portions of plate and then inserting a compression screw into a first portion of plate. This results in the compression of at least one fracture of bone. Thereafter, a compression screw can be inserted into second portion after removing the previously inserted locking or non-locking screw. This results in the compression of another fracture. Finally, a compression screw can be inserted into third portion after removing the previously inserted locking or non-locking screw to compress yet another fracture. Compression screws include non-locking screws.

In further detail, the method of stabilizing bone typically includes a mid-line posterior incision that exposes distal humerus medially, posteriorly, and laterally. This type of incision allows for insertion of bone plate. The method of stabilizing bone first includes taking scans of bone and evaluating which plate structure will be most efficient to stabilize bone and reduce bone fragments. The type of bone fracture, individual bone anatomy, and surgeon preference are considered when choosing bone plate structure. Plates would primarily be applied in complete articular elbow fractures (type 13C fractures as per AO/OTA classification). This includes simple articular, simple metaphyseal (type 13C1 as per AO/OTA classification), simple articular, fragmentary metaphyseal (type 13C2 as per AO/OTA classification) and multifragmentary articular metaphyseal (type 13C3 as per AO/OTA classification). In clinical practice, a specific fracture pattern may indicate a specific dual plate selection.

After bone anatomy and bone fracture are evaluated and an appropriate bone plate is determined, a suitable bone plate is positioned over bone fracture, for instance plate 100. The bone plate is fixed to one spot on bone surface using bone screws. Elongated holes 112 and non-locking holes 110 may receive bone screws in a non-fixed state allowing for final fixation and positioning of bone plate. Exact positioning of bone plate can be adjusted to achieve desired positioning and compression along bone surface for bone fragment reduction. Elongated holes 112 also allow for adjusting positioning and orientation of bone plate because bone plate may be moved along a distance of elongated opening before final screws are fixed into place.

After bone plate is fixed into place, multi-directional compression or decompression is applied along bone surface to reduce bone fragments by inserting screws into locking compression holes 108. Once reduction is complete, final locking and non-locking screws may be inserted into locking screw holes, non-locking holes 110, and elongated holes 112 to fix bone fragments into place. Locking screws can be angled within non-locking holes 110 and locking compression holes 108 *(e.g.,* up to 15 degrees in each direction for a total range of 30 degrees) depending upon patient anatomy.

Bone plate may be manufactured via an additive manufacturing process. Additionally, bone plates and bone screws may be made of a biocompatible material such as a polymer e.g., reinforced polymer composites; a titanium alloy e.g., Ti6Al4V; stainless steel e.g., 316L; or other metals and metal alloys, such as Ti and CoCr.

Unitary plate systems allow for a faster and easier application of bone plating to stabilize bone. Further, less drilled holes and screws are needed on bone along plate shaft in particular with the combination of non-locking holes and locking holes along bone plate that allow for exact positioning of bone plate before fixing it into place. Plates according to the present disclosure also allow for dual plating combinations that are not possible with dual plates such as a combination of posterolateral plates and posteromedial plates.

## Claims

1. A bone plate comprising:
a unitary plate (100) including a first portion (102), a second portion (104), and a third portion (106), each of the second and third portions being attached to the first portion and branching away from each other; and
at least one compression hole (108) on each portion of the unitary plate (100),
wherein the compression holes (108) are configured to allow compression along three different directions (114),
**characterized in that** the unitary plate (300) is configured to allow multi-directional compression along the three different directions (314) towards a single point.

2. The bone plate of claim 1, wherein the unitary plate (100) is Y-shaped.

3. The bone plate of claim 1 or claim 2, wherein at least one compression hole (108) is a locking compression hole.

4. The bone plate of any one of claims 1-3, further comprising a non-locking hole (110).

5. The bone plate of any one of claims 1-4, further comprising a locking hole.

6. The bone plate of any one of claims 1-5, wherein the compression hole (100) is configured to allow independent compression along the first portion (102), the second portion (104), and the third portion (106).

7. The bone plate of claim 3, wherein the locking compression hole is configured to accept a locking screw and a non-locking screw at varying angles with respect to a central axis of the hole.

8. The bone plate of any one of claims 1-7, wherein the second portion (104) and the third portion (106) branch away from each other at an angle of 90 or 180 degrees.

9. The bone plate of any one of claims 1-8, wherein the unitary plate (300) is configured to allow multi-directional compression along the three different directions (314).

10. The bone plate of any one of claims 1-9, wherein the unitary plate (300) is configured to allow multi-directional compression along the three different directions (314) at an angle of 50 to 160 degrees.

11. The bone plate of any one of claims 1-10, wherein the unitary plate (100) is configured to allow multi-directional selective compression and decompression along the three different directions (114, 118).

12. The bone plate of any one of claims 1-11, wherein the unitary plate (300) is configured to allow varying compression length along the three different directions (314).

13. The bone plate of any one of claims 1-12, wherein the unitary plate (700) is configured to allow lagging of bone fragments in a crisscross function.

14. The bone plate of any one of claims 1-13, wherein the unitary bone plate is additively manufactured.

## Patentansprüche

1. Knochenplatte, umfassend:
eine einteilige Platte (100) mit einem ersten Abschnitt (102), einem zweiten Abschnitt (104) und einem dritten Abschnitt (106), wobei der zweite und der dritte Abschnitt jeweils mit dem ersten Abschnitt verbunden sind und voneinander abzweigen; und
mindestens ein Kompressionsloch (108) an jedem Abschnitt der einteiligen Platte (100),
wobei die Kompressionslöcher (108) so ausgebildet sind, dass sie Kompression entlang dreier verschiedener Richtungen (114) ermöglichen,
**dadurch gekennzeichnet, dass** die einteilige Platte (300) so ausgebildet ist, dass sie multidirektionale Kompression entlang der drei verschiedenen Richtungen (314) auf einen einzigen Punkt hin ermöglicht.

2. Knochenplatte nach Anspruch 1, wobei die einteilige Platte (100) Y-förmig ist.

3. Knochenplatte nach Anspruch 1 oder Anspruch 2, wobei mindestens ein Kompressionsloch (108) ein Verriegelungskompressionsloch ist.

4. Knochenplatte nach einem der Ansprüche 1 bis 3, ferner umfassend ein Nicht-Verriegelungsloch (110).

5. Knochenplatte nach einem der Ansprüche 1 bis 4, ferner umfassend ein Verriegelungsloch.

6. Knochenplatte nach einem der Ansprüche 1 bis 5, wobei das Kompressionsloch (100) so konfiguriert ist, dass es unabhängige Kompression entlang des ersten Abschnitts (102), des zweiten Abschnitts (104) und des dritten Abschnitts (106) ermöglicht.

7. Knochenplatte nach Anspruch 3, wobei das Verriegelungskompressionsloch so ausgebildet ist, dass es eine Verriegelungsschraube und eine Nicht-Verriegelungsschraube in unterschiedlichen Winkeln in Bezug auf eine Mittelachse des Lochs aufnimmt.

8. Knochenplatte nach einem der Ansprüche 1 bis 7, wobei der zweite Abschnitt (104) und der dritte Abschnitt (106) in einem Winkel von 90 oder 180 Grad voneinander abzweigen.

9. Knochenplatte nach einem der Ansprüche 1 bis 8, wobei die einteilige Platte (300) so ausgebildet ist, dass sie multidirektionale Kompression entlang der drei verschiedenen Richtungen (314) ermöglicht.

10. Knochenplatte nach einem der Ansprüche 1 bis 9, wobei die einteilige Platte (300) so konfiguriert ist, dass sie multidirektionale Kompression entlang der drei verschiedenen Richtungen (314) in einem Winkel von 50 bis 160 Grad ermöglicht.

11. Knochenplatte nach einem der Ansprüche 1 bis 10, wobei die einteilige Platte (100) so konfiguriert ist, dass sie multidirektionale selektive Kompression und Dekompression entlang der drei verschiedenen Richtungen (114, 118) ermöglicht.

12. Knochenplatte nach einem der Ansprüche 1 bis 11, wobei die einteilige Platte (300) so konfiguriert ist, dass sie ein Variieren der Kompressionslänge entlang der drei verschiedenen Richtungen (314) ermöglicht.

13. Knochenplatte nach einem der Ansprüche 1 bis 12, wobei die einteilige Platte (700) so konfiguriert ist, dass sie ein Versetzen von Knochenfragmenten in einer Kreuzfunktion ermöglicht.

14. Knochenplatte nach einem der Ansprüche 1 bis 13, wobei die einteilige Knochenplatte additiv gefertigt ist.

## Revendications

1. Plaque vissée comprenant:
une plaque monobloc (100) comprenant une première partie (102), une deuxième partie (104) et une troisième partie (106), chacune des deuxième et troisième parties étant fixées à la première partie et s'éloignant l'une de l'autre; et
au moins un trou de compression (108) sur chaque partie de la plaque monobloc (100),
dans laquelle les trous de compression (108) sont conçus pour permettre une compression dans trois directions différentes (114),
**caractérisée en ce que** la plaque monobloc (300) est conçue pour permettre une compression multidirectionnelle le long des trois directions différentes (314) vers un point unique.

2. Plaque vissée selon la revendication 1, dans laquelle la plaque monobloc (100) a la forme d'un Y.

3. Plaque vissée selon la revendication 1 ou la revendication 2, dans laquelle au moins un trou de compression (108) est un trou de compression de verrouillage.

4. Plaque vissée selon l'une quelconque des revendications 1 à 3, comprenant en outre un trou sans verrouillage (110).

5. Plaque vissée selon l'une quelconque des revendications 1 à 4, comprenant en outre un trou de verrouillage.

6. Plaque vissée selon l'une quelconque des revendications 1 à 5, dans laquelle l'orifice de compression (100) est conçu pour permettre une compression indépendante le long de la première partie (102), de la deuxième partie (104) et de la troisième partie (106).

7. Plaque vissée selon la revendication 3, dans laquelle le trou de compression de blocage est conçu pour accueillir une vis de blocage et une vis sans blocage selon différents angles par rapport à l'axe central du trou.

8. Plaque vissée selon l'une quelconque des revendications 1 à 7, dans laquelle la deuxième partie (104) et la troisième partie (106) s'écartent l'une de l'autre en formant un angle de 90 ou de 180 degrés.

9. Plaque vissée selon l'une quelconque des revendications 1 à 8, dans laquelle la plaque monobloc (300) est conçue pour permettre une compression multidirectionnelle selon les trois directions différentes (314).

10. Plaque vissée selon l'une quelconque des revendications 1 à 9, dans laquelle la plaque monobloc (300) est conçue pour permettre une compression multidirectionnelle selon les trois directions différentes (314) à un angle allant de 50 à 160 degrés.

11. Plaque vissée selon l'une quelconque des revendications 1 à 10, dans laquelle la plaque monobloc (100) est conçue pour permettre une compression et une décompression sélectives multidirectionnelles selon les trois directions différentes (114, 118).

12. Plaque vissée selon l'une quelconque des revendications 1 à 11, dans laquelle la plaque monobloc (300) est conçue pour permettre une longueur de compression variable selon les trois directions différentes (314).

13. Plaque vissée selon l'une quelconque des revendications 1 à 12, dans laquelle la plaque monobloc (700) est conçue pour permettre le calage des fragments osseux selon un agencement entrecroisé.

14. Plaque vissée selon l'une quelconque des revendications 1 à 13, dans laquelle la plaque osseuse monobloc est fabriquée par fabrication additive.
